# EUROPÄISCHE PATENTSCHRIFT

(11) **EP 1 294 924 B1**
(45) Veröffentlichungstag und Bekanntmachung des Hinweises auf die Patenterteilung: **11.01.2006**
(21) Anmeldenummer: 01962692.8
(22) Anmeldetag: 30.05.2001
(51) Int. Cl.: C12Q 1/37, A61K 31/435, C07D 211/70

(54) **KOMPETITIONSVERFAHREN ZUM AUFFINDEN VON ALLOSTEREN INHIBITOREN**
COMPETITIVE ASSAY FOR IDENTIFYING ALLOSTERIC INHIBITORS
ESSAI COMPETITIF POUR L'IDENTIFICATION DES INHIBITEURS ALLOSTERIQUES

(30) Priorität: 09.06.2000 DE 10028204
(43) Veröffentlichungstag der Anmeldung: 26.03.2003
(73) Patentinhaber: Sanofi-Aventis Deutschland GmbH, 65929 Frankfurt am Main (DE)
(72) Erfinder: WEITHMANN, Klaus, Ulrich, 65719 Hofheim (DE)
(86) Internationale Anmeldenummer: PCT/EP2001/006143
(87) Internationale Veröffentlichungsnummer: WO 2001/094612

(56) Entgegenhaltungen:
- WO-A-90/12580
- US-A- 5 434 052
- STACK M S ET AL: "COMPARISON OF VERTEBRATE COLLAGENASE AND GELATINASE USING A NEW FLUOROGENIC SUBSTRATE PEPTIDE" JOURNAL OF BIOLOGICAL CHEMISTRY, Bd. 264, Nr. 8, 1989, Seiten 4277-4281, XP002198328 ISSN: 0021-9258
- DATABASE WPI Section Ch, Week 198849 Derwent Publications Ltd., London, GB; Class B04, AN 1988-353931 XP002198329 -& WO 88 09347 A (KABIVITRUM AB), 1. Dezember 1988 (1988-12-01)
- LAZERENO S ET AL: "ALLOSTERIC INTERACTIONS OF STAUROSPORINE AND OTHER INDOLOCARBAZOLESWITH N-METHYL-3HSCOPOLAMINE AND ACETYLCHOLINE AT MUSCARINIC RECEPTOR SUBTYPES: IDENTIFICATION OF A SECOND ALLOSTERIC SITE" MOLECULAR PHARMACOLOGY, BALTIMORE, MD, US, Bd. 58, Nr. 1, Juli 2000 (2000-07), Seiten 194-207, XP000997198 ISSN: 0026-895X
- 1975, BIOCHEMISTRY, LEHNINGER, A.L., WORTH PUBLISHERS, NY, USA
- THE JOURNAL OF BIOLOGICAL CHEMISTRY Bd. 278, Nr. 32, August 2003, Seiten 29901 - 29912
- MOLECULAR CELL Bd. 9, Juni 2002, Seiten 1241 - 1249
- THE JOURNAL OF BIOLOGICAL CHEMISTRY Bd. 276, Nr. 25, Juni 2001, Seiten 23105 - 23108
- THE JOURNAL OF BIOLOGICAL CHEMISTRY Bd. 278, Nr. 32, August 2003, Seiten 30136 - 30141
- EXPERT OPIN. THER. PATENTS Bd. 12, Nr. 5, 2002, Seiten 665 - 707

## Beschreibung

Die vorliegende Erfindung betrifft ein Verfahren zum Auffinden von neuartig und vorteilhaft wirkenden, medizinisch wertvollen Substanzen die Inhibitoren eines Proteins sind.

Enzyme sind hochmolekulare Proteine, die in allen Zellen vorkommen. Als Biokatalysatoren ermöglichen sie den Ablauf der zahlreichen biochemischen Stoffwechselwege des Lebens. Dabei sind sie jeweils für ganz bestimmte Reaktionen spezifisch. Sie treten mit Substraten in Wechselwirkung; als Zwischenstufe bildet sich ein Enzym/Substratkomplex, und schließlich wird nach chemischer Umwandlung das gebildete Produkt in das Medium abgegeben.

Einfache Enzyme tragen nur eine Bindungsstelle für die Aufnahme des Substrates. Komplexere Systeme bestehen aus mehreren kovalent oder nicht-kovalent verbundenen Enzymdomänen mit den Bindungsstellen A, B, C, D usw., die häufig jeweils einer Enzymdomäne zugeordnet werden. Diese Bindungsstellen erkennen wiederum im Substrat eine oder mehrere strukturelle bzw. chemisch funktionelle Gruppen Z, die gleich (Z1, Z2, Z3 usw.) oder verschieden sein können, d.h. Y, X, W usw..

Entsprechende komplexere Enzymreaktionen sind aus mehreren Teilreaktionen zusammengesetzt. So kann die Domäne A mit der Substratgruppe Z1 in Wechselwirkung treten. Die Domäne B kann ebenfalls mit Z1 reagieren, oder mit Z2, oder mit einer oder mehreren Stellen Y, X usw.

Eine katalytische Domäne ist eine Aminosäuresequenz eines Proteins oder Enzyms, die ein Substrat bindet und chemisch umwandelt. Eine Bindungsdomäne ist eine Aminosäuresequenz eines Proteins oder Enzyms, die ein Substrat reversibel bindet. In diesen enzymatischen Systemen können Bindungsstellen statt des Substrates auch einen oder mehrere Liganden erkennen und binden, die sich aber nicht wie ein Substrat verhalten, das heißt nicht chemisch umgesetzt werden, die sich aber auch nicht immer als Inhibitoren verhalten.

Wenn man Inhibitoren oder Liganden für komplexe Proteine sucht, werden in der Regel Inhibitoren für die katalytische Domäne gefunden, weil diese Inhibitoren eine Verringerung der chemischen Umsetzung des Substrates bewirken. Diese können auch durch die fehlenden oder verringerten Umsetzungen des Markersubstrats angezeigt werden. Wesentlich schwieriger ist es, Inhibitoren für die Bindungsdomäne zu finden.

Aufgabe der vorliegenden Erfindung ist es daher Stoffe zu finden, die die Bindung von Substraten an die Bindungsdomäne eines Proteins, verringern oder im wesentlichen verhindern. Dieser Stoff kann inhibitorisch wirken oder ein Ligand sein.

Die Aufgabe wird dadurch gelöst, dass ein Protein, das Markersubstrat und das Substrat mit einem Stoff inkubiert werden und festgestellt wird, ob das Markersubstrat vom Protein umgesetzt wird.

Die Erfindung betrifft daher ein Verfahren zur Bestimmung, ob ein Stoff ein Inhibitor oder ein Ligand eines Proteins ist, wobei man für eine Bindungsdomäne eines Proteins ist, wobei es sich bei der Bindungsdomäne um keine Katalytische Domäne handelt, wobei man
a) ein Protein einsetzt, welches mindestens eine katalytische Domäne enthält und mindestens eine Bindungsdomäne enthält,
b) mindestens ein Markersubstrat einsetzt, welches an die katalytische Domäne bindet und umgesetzt wird,
c) mindestens ein Substrat einsetzt, welches an die katalytische Domäne und an die Bindungsdomäne binden kann,
d) das genannte Protein, das Markersubstrat und das Substrat mit dem Stoff oder Liganden inkubiert und
e) bestimmt, ob das Markersubstrat vom Protein umgesetzt wird und
f) die Verfahrensschritte a) bis d) in Abwesenheit des Stoffes wieder holf und
g) die Hesswerte der Inkubationen in An-und Abwesenheit des Stoffes miteinander vergleicht.
Geeignete Proteine sind beispielsweise Enzyme, die mindestens eine katalytische Domäne und mindestens eine Bindungsdomäne enthalten. Die Enzyme können aber auch 1 bis 8 katalytische Domänen und 1 bis 8 Bindungsdomänen enthalten, bevorzugt sind jeweils 1, 2, 3 und 4 katalytische Domänen bzw Bindungsdomänen. Das Substrat ist eine Verbindung, die sowohl an die Bindungsdomäne als auch an die katalytische Domäne binden kann, wobei das Substrat von der katalytischen Domäne chemisch umgesetzt wird und von der Bindungsdomäne chemisch nicht verändert wird. Das Markersubstrat ist eine vom Substrat chemisch unterschiedliche Verbindung, die von der katalytischen Domäne chemisch umgesetzt wird und es erlaubt die Umsetzungsreaktion zu verfolgen.

Der Stoff bindet im wesentlichen reversibel oder irreversibel mit der Bindungsdomäne des Proteins und verhindert dadurch die Bindung des Substrats an das Protein. Der Stoff kann daher als Inhibitor der Bindungsdomäne des Proteins angesehen werden. Der Stoff verhindert im wesentlichen nicht die chemische Umwandlung des Markersubstrats durch die katalytische Domäne des Proteins.

Ein Beispiel für ein geeignetes Protein ist das Enzym Kollagenase, das aus mindestens zwei kovalent verbundenen Enzymdomänen besteht, einer Kollagen bindenden, sowie einer weiteren mit proteolytischen Fähigkeiten, die den Kollagenstrang durchschneidet. Diese katalytische proteolytische Domäne, sei es nun im kovalenten Verbund des Enzyms mit voller Länge, d.h. des natürlich vorkommenden aktiven Enzyms, oder nur die rekombinant hergestellte proteolytische Domäne, besitzt die Fähigkeit, auch verschiedenartige Markersubstrate zu spalten.

Geeignete Markersubstrate für die Kollagenase sind beispielsweise Peptide, die mit Fluoreszenz- oder UV-Markern versehen sind. Beispiele für Markersubstrate für derartige Enzyme sind (7-Methoxycoumarin-4-yl)-acetyl-Pro-Leu-Gly-Leu-(3-[2,4-dinitrophenyl]-L-2,3-di-aminopropionyl)-Ala-Arg-NH₂, (Knight, C.G., et al, FEBS Letters (1992) 296, 263-266), Dnp-Pro-β-cyclohexyl-Ala-Gly-Cys(Me)-His-Ala-Lys(N-Me-Abz)-NH₂ (Bickett D. M., et al., Analytical Biochemistry (1993) 212, 58-64); Mca-Pro-Cha-Gly-Nva-His-Ala-Dpa-NH₂ (Knäuper V., et al., JBC (1996) 271/3, 1544-1550); Mca-Arg-Pro-Lys-Pro-Val-Glu-Nva-Trp-Arg-Lys-Dnp-NH₂ (Nagase H., et al., JBC (1994) 269/33, 20952-20957); Dnp-Arg-Pro-Lys-Pro-Leu-Ala-Nva-Trp-NH₂ (Niedzwiecki L., et al., Biochemistry (1992) 31, 12618-12623) (Bickett D. M., et al., Analytical Biochemistry (1993) 212, 58-64; Knight, C.G., et al, FEBS Letters (1992) 296, 263-266; Knäuper V., et al., *JBC* (1996) 271/3, 1544-1550; Nagase H., et al., JBC (1994) 269/33, 20952-20957; Niedzwiecki L., et al., Biochemistry (1992) 31, 12618-12623) oder radioaktiv markierte Peptide.

Ein geeignetes Substrat für die Kollagenase ist Kollagen. Kollagen ist ein prolinreicher Gerüsteiweißkörper (Skleroprotein) der ein gegen enzymatische Angriffe schützender Hauptbestandteil mesenchymaler interzellulärer Stützsubstanzen ist. Drei Eiweißketten mit linksläufiger Helixstruktur sind zu einer rechtsdrehenden Tripelhelix (Superhelix) verdrillt. Identifiziert sind 18 Kollagen-Typen (Kollagen-Typ I bis Typ XVIII), die nach Struktur bzw. Funktion in fibrilläre, Fibrillen-assoziierte und nichtfibrilläre Kollagene eingeteilt werden.

Besonders geeignet ist Kollagen vom Typ II als Substrat.
Dieses Kollagen stützt die Knorpelmatrix in Gelenken. In gewissen Krankheiten wie Osteoarthritis und Rheuma findet eine Zerstörung des Gelenkes statt, besonders bedingt durch den proteolytischen Abbau von Kollagen durch Kollagenasen. Inhibitoren derartiger Enzyme sind bekannt, haben aber den Nachteil, daß sie an der katalytischen Domäne des Enzyms angreifen (Weithmann K. U., et al., Inflamm. Res. (1997) 46, 246-252). Diese katalytische Domäne ist in ähnlicher Struktur vielen Enzymen zu eigen, demzufolge wirken die Inhibitoren in unerwünschter Weise auf viele Enzyme, auch solche mit vitaler Funktion ein (Massova I., et al., The FASEB Journal (1998) 12, 1075-1095). Aufgabe der vorliegenden Erfindung ist es der Medizin bessere Inhibitoren mit höherer Spezifität auf die jeweilen Enzyme zur Verfügung zu stellen.

Inkubiert man Markersubstrat, Kollagen Typ II mit dem Enzym Kollagenase und bestimmt den Umsatz des Markersubstrats, so stellt man überraschenderweise fest, dass der Umsatz des Markersubstrats stark inhibiert wird. Diese Inhibition kann nur durch hohe Mengen vom Markersubstrat überwunden werden. Diese Hemmung des Umsatzes des Markersubstrats tritt jedoch nicht auf, wenn nur die katalytische Domäne von der Kollagenase mit Kollagen II und Markersubstrat inkubiert werden.

Überraschenderweise können bei dem erfindungsgemäßen Verfahren Stoffe identifiziert werden, die die Hemmung der Umsetzung vom Markersubstrat im wesentlichen aufheben, wenn die vollständige Kollagenase mit Markersubstrat, Kollagen Typ II und dem Stoff inkubiert werden. Dieser Effekt tritt nicht auf, wenn statt der vollständigen Kollagenase nur die katalytische Domäne der Kollagenase eingesetzt wird.

Enzyminhibitoren gemäß Stand der Technik (Weithmann K. U., et al., Inflamm. Res. (1997) 46, 246-252) bewirken demgegenüber eine verstärkte Hemmung der Umsetzung von Markersubstrat, sowohl katalysiert durch das vollständige Enzym als auch durch die katalytische Domäne.

Beispiele für den Begriff "Protein, welches mindestens eine Bindungsdomäne und mindestens eine katalytische Domäne enthält" sind die Mitglieder der Matrixine , die Enzyme Gelatinase, Kollagenase-1 , Neutrophilen-Kollagenase oder Matrix-Metalloproteinase Typ 13 (Massova I., et al., The FASEB Journal (1998) 12, 1075-1095).

Das Auffinden eines erfindungsgemäßen Stoffes wird dadurch angezeigt, dass im erfindungsgemäßen Verfahren, das Markersubstrat mit höherer Umsatzrate vom Protein umgesetzt wird. Im Gegensatz zu Inhibitoren gemäß Stand der Technik inhibieren die erfindungsgemäßen Stoffe nicht die enzymatische Domäne, sondern sie interferieren mit der Bindung des Substrates an die Bindungsdomäne(n) des Enzyms. Die erfindungsgemäßen Stoffe bewirken eine Inhibierung mit verbesserter Spezifität, da sie nicht an der Enzymdomäne, die in vielen Enzymtypen vorkommt, angreifen.

In einem bevorzugten Verfahren werden als Protein Kollagenase, als Substrat Kollagen Typ II und (7-Methoxycoumarin-4-yl)-acetyl-Pro-Leu-Gly-Leu-(3-[2,4-dinitrophenyl]-L-2,3-di-aminopropionyl)-Ala-Arg-NH₂ als Markersubstrat eingesetzt.

Gegenstand der Erfindung ist ferner ein Testkit zur Durchführung des erfindungsgemäßen Verfahrens, der aus den Bestandteilen
A) ein Protein, welches mindestens eine katalytische Domäne und mindestens eine Bindungsdomäne hat,
B) ein Markersubstrat, welches an die katalytische Domäne bindet und umgesetzt wird, und
C) ein Substrat, welches an die katalytische Domäne und an die Bindungsdomäne binden kann, besteht.

Es wurde nun gefunden, daß der durch das erfindungsgemäße Verfahren aufgefundene Stoff 4-(Diphenylmethylen)-1-[4-(p-fluorphenyl)-4-phenyl-3-butenyl] piperidin ein starker Inhibitor der Metalloproteinasen ist. Dabei wird auf die Hemmung von Stromelysin (Matrix Metalloproteinase 3), der Neutrophilen Kollagenase (MMP-8) und der Aggrecanase besonderer Wert gelegt, da diese Enzyme beim Abbau der Proteoglykane, als wichtige Bestandteile des Knorpelgewebes, maßgeblich beteiligt sind (A. J. Fosang et al. J. Clin. Invest. 98 (1996) 2292-2299).

### Beispiele

### Herstellung der BESTANDTEILE:

### BESTANDTEIL A)

### Enzym-Lösung:

MMP-13 wird als Handelsprodukt (Kat.Nr. HM 13110010) von Invitek GmbH, Berlin, Deutschland erhalten, nach Herstelleranweisung aktiviert, und mit TCB-Puffer auf 10µg/10ml aufgefüllt. TCB-Puffer wird hergestellt, indem10 mM Tris-(hydroxymethyl)-aminomethan (Sigma-Aldrich Chemie GmbH, Deisenhofen, Deutschland) in Wasser gelöst und mit HCl auf einen pH von 7,5 eingestellt werden. Zu dieser Lösung werden 100 mM CaCl₂x2H₂O (Merck KGaA, Darmstadt, Deutschland) und 0,05 % Briij 35-Lösung, 30 % (w/v) (Sigma-Aldrich Chemie GmbH, Deisenhofen, Deutschland) zugefügt.

### BESTANDTEIL B)

### Markersubstrat-Lösung

Eine Lösung von 10 mmol/L (7-Methoxycoumarin-4-yl)-acetyl-Pro-Leu-Gly-Leu-(3-[2,4-dinitrophenyl]-L-2,3-diaminopropionyl)-Ala-Arg-NH₃ (Bachem Biochemica GmbH, Heidelberg, Deutschland) in Dimethylsulfoxid (DMSO) (Riedel-de Haen AG, Seelze, Deutschland) wird mit Wasser 1 : 40 (v/v) verdünnt.

### BESTANDTEIL C)

### Kollagen II-Lösung

10 mg humanes Kollagen II (Fa. Biocon, Potsdam, Deutschland) werden in 2400 µl 10mmol/L Essigsäure gelöst (72 Stunden bei 4° C) und dann tropfenweise mit 1300µl einer Natriumhydrogencarbonatlösung (250 mmol/L) CaCl₂x2H₂O (Merck KGaA, Darmstadt, Deutschland) versetzt.

### BESTANDTEIL D)

Erfindungsgemäßer Inhibitor, vorzugsweise gelöst in Wasser.

### Untersuchungsprotokoll

Die Bestandteile 25µl von A), 5µl von B), 10µl von C) und 10µl von D) werden im Gesamtvolumen von 50µl gemischt, und die Fluoreszenz in einem handelsüblichen Spektrofluorimeter nach 15 Minuten gemessen (Anregung bei 330nm, Emission bei 390 nm, Spectrafluor plus, Tecan Deutschland GmbH, Crailsheim, Deutschland)).

Enzym und Inhibitor werden für 15 min bei Raumtemperatur vorinkubiert. Die Reaktion wird durch Zusatz von 5 µl Markersubstrat-Lösung BESTANDTEIL B) (25 µM) gestartet.

Die Fluoreszenz im Beispiel 1A wurde auf 100% gesetzt, und die Meßwerte der weiteren Beispiele auf diesen Wert bezogen.

**Tabelle 1 zeigt die Ergebnisse.**

| | Beispiel 1 | Beispiel 2 | Beispiel 3 | Beispiel 4 | Beispiel 5 | Beispiel 6 |
|---|---|---|---|---|---|---|
| Bestandteil A | ja | ja | ja | ja | ja | ja |
| Bestandteil B | ja | ja | ja | ja | ja | ja |
| Bestandteil C | nein | ja | ja | ja | ja | ja |
| Bestandteil D | nein | nein | 200µmol/L Stoff (I) | 200µmol/L Doxycycli n | 50µg/well Tetrasaccharide | 60µg/well Protaminsulfat |
| Meßwert | 100% | 49% | 59% | 0% | 72% | 73% |

Beispiele 3, 5 und 6 entsprechen dem erfindungsgemäßen Verfahren, welches das Auffinden von neuartigen Inhibitoren oder Liganden, ermöglicht. Diese sind dadurch gekennzeichnet, daß sie in einer Konzentration bis 500 µmol/L, vorzugsweise 50 µmol/L oder kleiner, insbesondere auch schon ab 1 nmol/L, im Ausnahmefall bis 0,1 nmol/L, den Fluoreszenzwert von 50-99% ergeben.

### Beispiel 3:

200µmol/L des erfindungsgemäßen Stoffes (I) 4-(Diphenylmethylen)-1-[4-(p-fluorphenyl)-4-phenyl-3-butenyl] piperidin 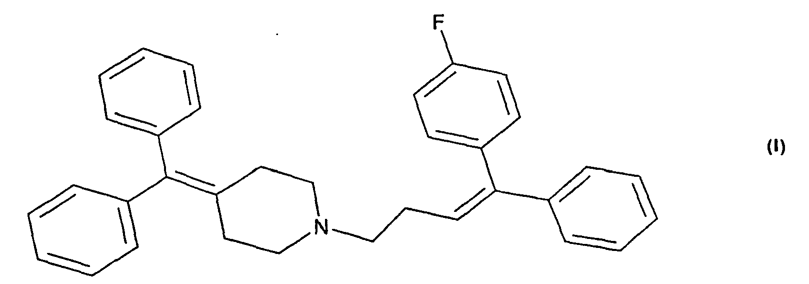 ergeben den Wert 59%.

### Beispiel 4

Davon abzugrenzen sind solche Stoffe, die Meßwerte von 49% und kleiner, bis auf Null absinkend ergeben. Entsprechend dem Stand der Technik ergeben 200µmol/L Doxycyclin (Weithmann K. U., et al., Inflamm. Res. **(1997)** 46, 246-252) den Wert 0.

### Beispiel 5

50µg/50µL Tetrasaccharide GT8021 (Neoparin,Inc., 14274 Wicks Blvd, San Leandro, CA 94577). Erfindungsgemäß ergeben 50µg/50µl Tetrasaccharide den Wert 72 %.

### Beispiel 6

60µg/50µL Protaminsulfat P-4020 (Sigma-Aldrich Chemie GmbH, Deisenhofen, Deutschland). Erfindungsgemäß ergeben 60µg/50µl Protaminsulfat den Wert 73 %.

## Patentansprüche

1. Verfahren zur Bestimmung, ob ein Stoff ein Inhibitor oder ein Ligand für eine Bindungsdomäne eines Proteins ist, wobei es sich bei der Bindungsdomäne um keine katalytische Domäne handelt, wobei man
a) ein Protein einsetzt, welches mindestens eine katalytische Domäne enthält und mindestens eine Bindungsdomäne enthält,
b) mindestens ein Markersubstrat einsetzt, welches an die katalytische Domäne bindet und umgesetzt wird,
c) mindestens ein Substrat einsetzt, welches an die katalytische Domäne und an die Bindungsdomäne binden kann,
d) das genannte Protein, das Markersubstrat und das Substrat mit dem Stoff inkubiert und
e) bestimmt, ob das Markersubstrat vom Protein umgesetzt wird und
f) die Verfahrensschritte a) bis d) in Abwesenheit des Stoffes wiederholt und
g) die Messwerte der Inkubationen in An- und Abwesenheit des Stoffes miteinander vergleicht.

2. Verfahren gemäß Anspruch 1, **dadurch gekennzeichnet, dass** als Protein Kollagenase, als Substrat Kollagen und (7-Methoxycoumarin-4-yl)-acetyl-Pro-Leu-Gly-Leu-(3-[2,4-dinitrophenyl]-L-2,3-diaminopropionyl)-Ala-Arg-NH₂ als Markersubstrat eingesetzt werden.

## Claims

1. A method to determine whether a substance is an inhibitor or a ligand of a binding domain of a protein, the binding domain not being a catalytic domain, which comprises
a) using a protein which contains at least one catalytic domain and at least one binding domain,
b) using at least one marker substrate which binds to the catalytic domain and is converted,
c) using at least one substrate which can bind to the catalytic domain and to the binding domain,
d) incubating said protein, the marker substrate and the substrate with the substance, and
e) determining whether the marker substrate is converted by the protein,
f) repeating the method steps a) to d) in the absence of the substance, and
g) comparing the measured values of the incubations in the presence and absence of the substance.

2. The method as claimed in claim 1, wherein the protein used is collagenase, the substrate used is collagen, and the marker substrate used is (7-methoxycoumarin-4-yl)acetyl-Pra-Leu-Gly-Leu-(3-[2,4-dinitrophenyl]-L-2,3-diaminopropionyl)-Ala-Arg-NH₂.

## Revendications

1. Procédé pour déterminer si une substance est un inhibiteur ou un ligand pour un domaine de liaison d'une protéine dans lequel, pour ce qui concerne le domaine de liaison, il ne s'agit pas d'un domaine catalytique, dans lequel
a) on met en oeuvre une protéine qui contient au moins un domaine catalytique et au moins un domaine de liaison,
b) on met en oeuvre au moins un substrat marqueur qui se lie au domaine catalytique et est amené à réagir,
c) on met en oeuvre au moins un substrat qui peut se lier au domaine catalytique et au domaine de liaison,
d) on met à incuber la protéine citée, le substrat marqueur et le substrat avec la substance, et
e) on détermine si le substrat marqueur de la protéine est amené à réagir, et
f) on répète les étapes a) à d) du procédé en l'absence de la substance, et
g) on compare la valeur de mesure des incubations en présence et en l'absence de la substance.

2. Procédé suivant la revendication 1, **caractérisé en ce qu'**on met en oeuvre, comme protéine, de la collagénase, comme substrat, du collagène et, comme substrat marqueur, du (7-méthoxycoumarin-4-yl)-acétyl-Pro-Leu-Gly-Leu-(3-[2,4-dinitrophényl]-L-2,3-diaminopropionyl)-Ala-Arg-NH₂.
